# EUROPEAN PATENT SPECIFICATION

(11) **EP 3 111 844 B1**
(45) Date of publication and mention of the grant of the patent: **25.10.2017**
(21) Application number: 16171239.3
(22) Date of filing: 25.05.2016
(51) Int. Cl.: A61B 5/15, A61B 5/151

(54) **HIGH SPEED LANCING DEVICE WITH LANCET EJECTION ASSEMBLY**
HOCHSCHNELLE LANZETTENVORRICHTUNG MIT LANZETTEN AUSSTOSSANORDNUNG
DISPOSITIF AUTOPIQUEUR À GRANDE VITESSE AVEC UN ENSEMBLE D'ÉJECTION DE LANCETTE

(30) Priority: 02.07.2015 TW 104121568; 02.07.2015 TW 104210725
(43) Date of publication of application: 04.01.2017
(73) Proprietor: Joinsoon Medical Technology Co., Ltd., New Taipei City 221 (TW)
(72) Inventor: LEE, Jen-Fang, 221 New Taipei City (TW); HUANG, Chin-Chi, 265 Yilan County (TW)
(74) Representative: Huang, Chongguang

(56) References cited:
- EP-A1- 1 219 242
- TW-A- 201 103 500
- US-A1- 2004 254 599
- US-A1- 2012 143 086

## Description

### FIELD

The present disclosure generally relates to a high speed lancing device with lancet ejection assembly. Said lancing device can be applied for blood sampling at homes, or clinics and hospitals. The present disclosure can be applied to self-monitoring of glucose for individual patients, or glucose tests in clinics or hospitals.

### BACKGROUND

Monitoring of blood glucose level is essential for diabetes patients to manage their health. Unfortunately, such process requires puncturing the patient's skin for the collection of blood samples. Lancing devices are used to puncture the skin to obtain traces amount of blood. The blood sample is then applied to a glucose test strip to determine the glucose level. Through this process, diabetes patient can effectively monitor their diet and adjust their blood glucose levels.

Monitoring blood glucose levels is imperative for patients suffering from diabetes and must be performed on a daily basis. To accurately monitor the blood glucose concentration levels, patients would often be required to collect blood samples 3 to 4 times day, leaving multiple puncture wounds that may take a few days to recover and restore to its normal sensation. The pain associated with such frequent punctures not only causes physical discomfort but also psychological hardship. Therefore, an intuitively designed lancing device that inflicts minimal discomfort when used on a daily basis is very much needed.

Many reusable at-home blood glucose meters are commercially available. These meters with lancing devices are mostly mechanical devices with built-in springs. U.S. Patent No. 4,653,513, EP 1970007 and TWI 200533319 have disclosed several types of lancing mechanisms with built-in springs. It may comprise a lancet support member, a lancet that can be installed on a support member, and one or more built-in springs that are coupled to the lancet support member. When movement of the lancet support member is triggered by the built-in spring, the lancet installed on the lancet support member will puncture the skin.

The elasticity of the built-in spring from the above mentioned lancing device is incapable of dissipating immediately, even after the spring retracts to its pre-trigger position. Therefore, once the spring is activated, it will bounce back-and-forth triggering the lancet support member multiple times, thereby inflicting additional small cuts or puncture wounds on the skin. These multiple wounds created by the residual elasticity of the built-in spring not only increase the patient's pain but is entirely unnecessary.

Disposable single-use lancing devices are commonly used in hospitals and clinics. While aiming the sharp front end of the device at the puncture site, the user can press down on the rear end to trigger the spring movement of the lancet to puncture the skin.

TWI 201103500 has disclosed a single-use lancing device comprising a chuck member, upper cap, lancet support member, upper spring and lancet. The upper cap serves as the actuating member of the device. When the upper cap is pressed, the upper spring that is coupled to the upper cap will trigger the chunk member, thereby releasing the upper spring from its compressed state to a relaxed state. The heavier chunk member will collide with and eject the lighter lancet support member causing the coupled lancet to puncture the targeted area.

The single-use lancing device has an upper cap that is protruding outwards such that the user might unintentionally trigger the lancing device simply by touching the upper cap. Since the single-use lancing device is designed to be triggered only once, the device will be rendered useless after the unintentional trigger. Unintentional triggers may also inflict one or more puncture wounds to non-targeted areas of the skin.

The architecture of the multiple-use lancing device described above is in need of an improvement. The device presents an issue with the built-in spring inflicting repetitive puncturing wounds on the patient's skin caused by the back-and-forth movement of the lancet. The pain inflicted on the patient is thereby unnecessary increased due the repetitive puncturing.

The architecture of the single-use lancing devices described above is also in need of an improvement. The protruding location of the actuating member of the device causes unintentional triggers of the lancet. The unintentional triggers not only create waste by rendering the device useless, it causes multiple puncture wounds on non-targeted areas.

### SUMMARY

A high-speed lancing device with lancet ejection assembly, comprising: a housing for the user to hold, a lancet support assembly; a lancet that can be attached to said lancet support assembly; a recovery assembly, said recovery assembly comprises a sleeve and a first flexible element, said sleeve is on one side of said housing, said sleeve can engage said lancet support assembly; a loading assembly, said loading assembly comprises an impact assembly and a rear assembly, said impact assembly and said rear assembly are positioned on the longitudinal axis of said lancing pen, said impact assembly further comprises a rod and a second flexible element; a trigger element, said trigger element is at the side of said housing; a lancet ejection assembly, said lancet ejection assembly can remove said lancet from said lancet support assembly.

### BRIEF DESCRIPTION OF THE DRAWINGS

The present description will be better understood from the following detailed description read in light of the accompanying drawings, where:
Fig. 1 is a side view of the lancing device in accordance with the present disclosure.
Fig. 2 is a cross-sectional side view of the lancing device of Fig. 1 in accordance with the present disclosure.
Fig. 3 is an exploded view of the lancing device of Fig. 1 in accordance with the present disclosure.
Fig. 4 is a cross-sectional side view of another lancing device in accordance with the present disclosure.
Fig. 5 is an exploded view of the lancing device of Fig. 4 in accordance with the present disclosure.
Fig. 6 is a partial cross-sectional side view of the first state of the lancing device of Fig. 1 in accordance with the present disclosure.
Fig. 7 is a partial cross-sectional side view of the second state of the lancing device of Fig. 1 in accordance with the present disclosure.
Fig. 8 is a partial cross-sectional side view of the third state of the lancing device of Fig. 1 in accordance with the present disclosure.
Fig. 9 is a partial cross-sectional side view of the fourth state of the lancing device of Fig. 1 in accordance with the present disclosure.
Fig. 10 is a perspective view of the lancet support assembly, the sleeve and the rod in the first state of the lancing device of Fig. 1 in accordance with the present disclosure.
Fig. 11 is a perspective view of the relative positioning of the lancet support assembly, the sleeve and the rod in the second state of the lancing device of Fig. 1 in accordance with the present disclosure.
Fig. 12 is a perspective view of the relative positioning of the lancet support assembly, the sleeve and the rod in the third state of the lancing device of Fig. 1 in accordance with the present disclosure.
Fig. 13 is a perspective view of the relative positioning of the lancet support assembly, the housing and the lancet ejection assembly in the lancing device of Fig. 1 in accordance with the present disclosure.

### DETAILED DESCRIPTION

The present disclosure is directed to a high-speed lancing device. The trigger member of said lancing device is located on the outer side of the housing of said lancing device. Said trigger member is capable of triggering the impact assembly inside said housing to collide with the lancet support assembly, so that the lancet on said lancet support assembly will puncture the skin at high speed to reduce the pain felt by the patient.

The present disclosure is directed to a high speed lancing device comprising a housing. Said housing comprises a front end that is closer to the patient's skin and a rear end located distantly from the patient's skin. Said lancing device comprises a lancet support assembly, and said lancet support assembly comprises at least two engaging parts. Said lancing device comprises a lancet, and said lancet can be removably installed on said lancet support assembly. Said lancing device comprises a recovery assembly, and said recovery assembly comprises a sleeve and a first flexible element. Said sleeve is located on said front end of said housing, and said sleeve can be coupled to said at least two engaging parts of said lancet support assembly. Said first flexible element is located on the outer side of said sleeve. Said lancing device further comprises a loading assembly, and said loading assembly is partially covered by said sleeve. Said loading assembly comprises a rear assembly and an impact assembly. Said impact assembly comprises a first end and a second end, and said rear assembly is coupled to said first end of said impact assembly. Said impact assembly and said rear assembly are positioned on the longitudinal axis of said lancing device. Said impact assembly further comprises a rod and a second flexible element. Said lancing device further comprises a trigger member, and said trigger member is located on the side of said housing. Said impact assembly can be loaded by said rear assembly and configured to a pre-triggered position. Said trigger member can activate said impact assembly into a triggered position for colliding with said lancet support assembly. After being collided with said impact assembly, said lancet support assembly will move toward the direction of the patient's skin. Said lancet installed on said lancet support assembly will puncture the skin. The present disclosure is able for said lancet support assembly to move faster than said impact assembly after being collided with said impact assembly, therefore the puncturing time for said lancet is reduced and the pain felt by the patient is also reduced.

The high speed lancing device provided by the present disclosure further comprises a rear assembly. Said rear assembly comprises a third flexible element and a rear member, whereas said rear member and said third flexible element are positioned on the longitudinal axis of said lancing device. Said rear member partially covers said third flexible member. Said rear assembly can be pulled backwardly, thereby compressing said third flexible member. The pulling of said rear assembly causes the impact assembly to be configured to a pre-triggered position. Said pre-triggered position can be configured into a triggered state by the trigger member. The present disclosure prevents the user from unexpectedly triggering said trigger member of said lancing device to actuate said lancing device. Non-target sites on the skin of the patient are thus prevented from being wounded.

The mass of the impact assembly of the high speed lancing device provided by the present disclosure is larger than the mass of the lancet support assembly. Said impact assembly will collide with said lancet support assembly at a lower speed, in comparison with the higher speed of the movement of said lancet support assembly after being collided with said impact assembly. Because the mass of said lancet support assembly is smaller, so said lancet support assembly will be moved at a higher speed after being collided with said impact assembly. The present disclosure reduces puncturing time of said lancet, thus reducing the pain felt by the patient.

The high speed lancing device provided by the present disclosure further comprises a chunk member. Said chunk member is located on one end of the impact assembly and coupled to the rod. Said chunk member will directly contact with the lancet support assembly when it collides with said lancet support assembly. Said chunk member has a larger mass than the mass of said lancet support assembly. Said chunk member may be one or more metal chunks, or one or more plastic chunks mixed with metal particles. Because the mass of said lancet support assembly is smaller, so said lancet support assembly will be moved in a higher speed after being collided with said chunk member. The present disclosure reduces puncturing time of said lancet, thus reducing the pain felt by the patient.

The high speed lancing device provided by the present disclosure further comprises a lancet ejection assembly. Said lancet ejection assembly is located on the side of the housing, whereas said lancet ejection assembly is positioned through said housing to contact with the lancet support assembly. Said lancing device provided by the present disclosure can be used multiple times due to said lancet ejection assembly and the lancets that can be removably installed.

The high speed lancing device provided by the present disclosure further comprises a front assembly. Said front assembly is located on the front end of the housing. Said front assembly can be used to adjust the depth of skin puncture by the lancet. The user can control the depth of skin puncture via said front assembly in said lancing device provided by the present disclosure

The present disclosure is further directed to a method to obtain blood samples by puncturing skin. Said method comprising: (i) placing the end of the lancing device of the present disclosure that is close to the patient's skin to a desired puncture site on the skin, and aiming said lancing device at the desired puncture site on the skin, and contacting said lancing device to said desired puncture site; (ii) pulling the rear assembly of the loading assembly in said lancing device, so that said rear assembly is moved backwards to compress the second flexible element of said impact assembly, then the rod of said impact assembly is moved toward the rear end of the housing, and said rod contacts with the trigger member; (iii) pressing said trigger member, so that the second flexible element is deformed from a compressed state into a relaxed state, and said lancet support assembly is moved, then the first element is compressed and the puncturing end of the lancet on said lancet support assembly punctures the skin; (iv) after said puncturing end of said lancet punctures the skin, said first flexible element is deformed from a compressed state into a relaxed state, and said lancet support assembly is moved toward said rear end of said housing and engages the sleeve. The present disclosure ensures said lancet support assembly has a faster movement than said impact assembly. Therefore, the puncturing time is reduced and the pain felt by the patient is also reduced.

The present disclosure is directed to a high speed lancing device. Said lancing device uses the trigger member located on the outer side of said lancing device to activate the impact assembly. The lancet support assembly is then collided with said impact assembly, causing rapid movement of said lancet support. The lancet installed on said lancet support assembly will puncture the skin at high speed. The pain felt by the patient is reduced and unexpected triggering of lancing device is prevented by the above mentioned mechanism utilized by said lancing device. "The user" hereby refers to the person who uses the lancing device. "The patient" hereby refers to the person who will receive skin puncture in order to obtain blood samples. The user and the patient can be the same person.

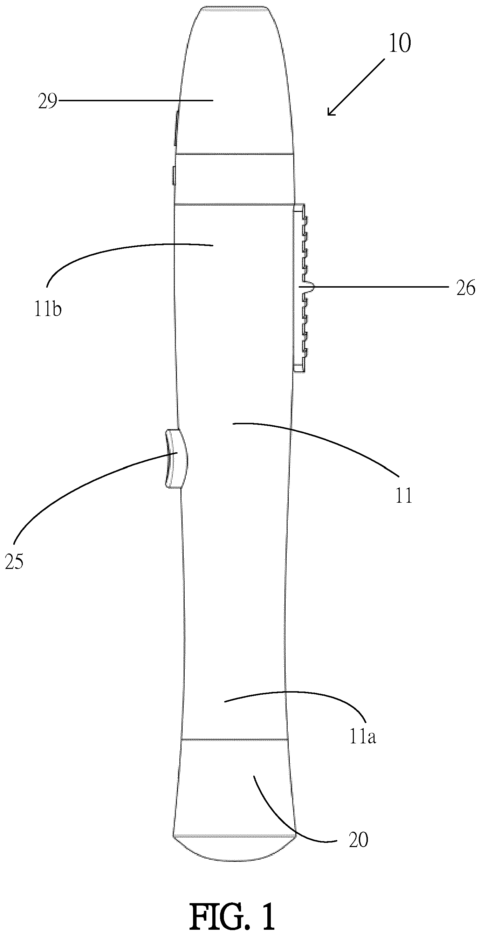

Fig. 1 is an illustration of the lancing device provided by the present disclosure. The lancing device 10 comprises a housing 11 that can be held by the user. Said housing 11 has a rear end 11a located at a position that is distant from the patient's skin, and a front end 11b located at a position that is closer to the patient's skin. A rear member 20 is coupled to said rear end 11a of said housing 11. A front assembly 29 is coupled to said front end 11b of said housing 11. A trigger member 25 and a lancet ejection assembly 26 are located on the side of said housing 11. Said lancing device 10 has a longitudinal axis, whereas said longitudinal axis is the direction extending from said rear member 20 to said front assembly 29.

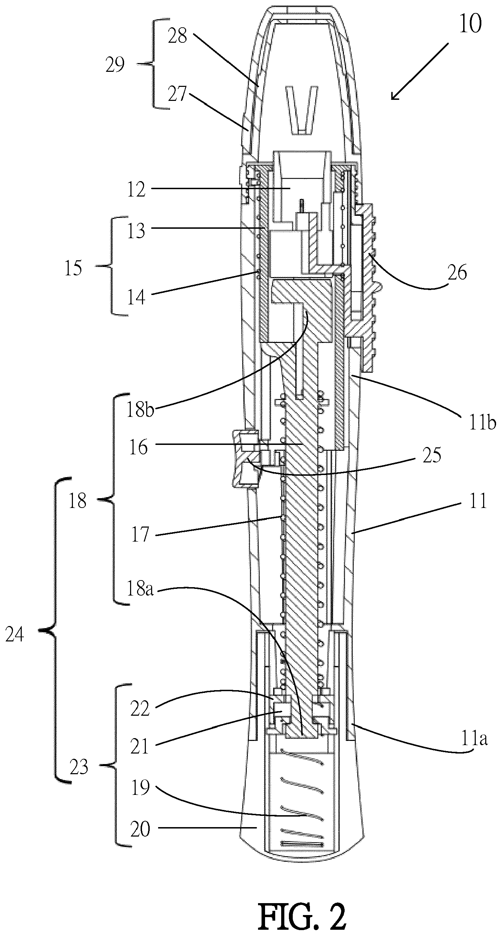

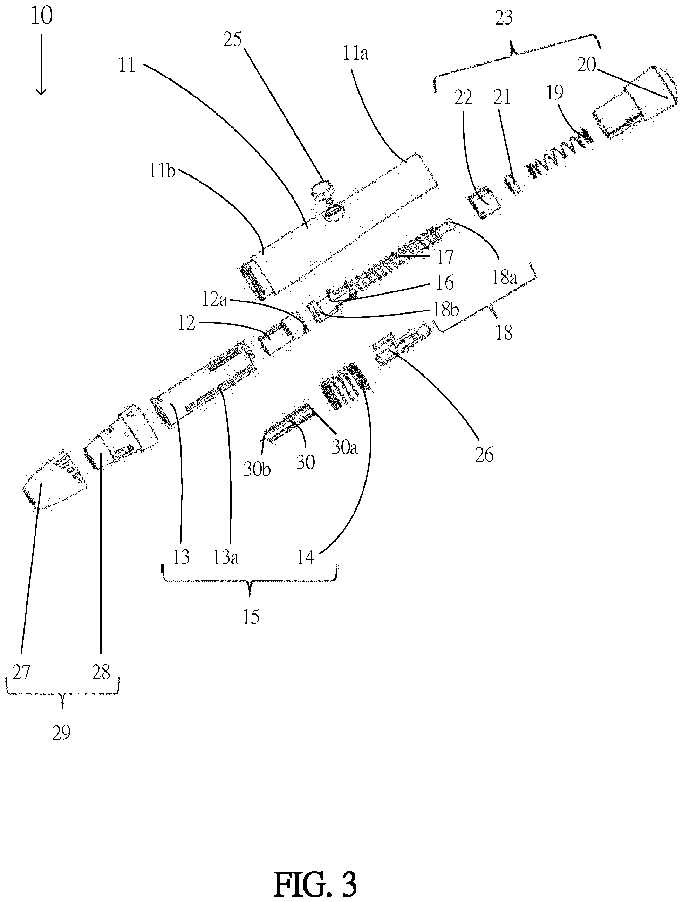

Referring to Fig. 2 and Fig. 3, Fig. 2 is a cross sectional side view and Fig. 3 is an exploded view of the lancing device 10 provided by the present disclosure. Said lancing device 10 comprises a housing 11 that can be held by the user. Said housing 11 further comprises a rear end 11a located at a position that is distant from the patient's skin, and a front end 11b located at a position that is closer to the patient's skin.

The lancing device 10 further comprises a lancet support assembly 12, whereas said lancet support assembly 12 is partially covered by the housing 11.

The lancing device 10 further comprises a recovery assembly 15, whereas said recovery assembly 15 is located on the front end 11b of the housing 11 and is partially covered by said housing 11. Said recovery assembly comprises a sleeve 13 and a first flexible element 14, and said sleeve 13 can engage with the lancet support assembly 12. Said first flexible element 14 is located on the outer side of said sleeve 13 and is partially covered by said housing 11.

The lancing device 10 further comprises a loading assembly 24, and said loading assembly 24 is partially covered by the housing 11. Said loading assembly 24 further comprises a rear assembly 23 positioned on the longitudinal axis of said lancing device 10, and an impact assembly 18. Said impact assembly 18 comprises a first end 18a located at a position that is distant from the patient's skin, and a second end 18b located at a position that is closer to the patient's skin. Said rear assembly 23 is coupled to said first end 18a of said impact assembly 18. Said impact assembly 18 further comprises a rod 16 positioned on the longitudinal axis of said lancing device 10, and a second flexible element 17. Said rear assembly 23 comprises a rear member 20 that can be pulled by the user. Said rear assembly 23 further comprises a third flexible element 19 and a support member 21, and said support member 21is used to position said first end 18a of said impact assembly 18 in said lancing device 10. Said rear assembly further comprises a fixing member 22. Said rear member 20 and said third flexible element 19 is positioned on the longitudinal axis of said lancing device 10.

The lancing device 10 further comprises a trigger member 25. Said trigger member 25 is located on the side of said housing.

The lancing device 10 further comprises a lancet 30 that can be removably installed on said lancet support member 12, and a lancet ejection assembly 26 located on the side of said housing 11.

The lancing device 10 further comprises a front assembly 29. Said front assembly 29 is used to adjust the depth of skin puncture made by the lancet 30. Said front assembly 29 comprises an outer front member 27 and an inner front member 26. Said outer front member 27 can be adjusted by the user for adjusting the depth of skin puncture made by said lancet 30.

Before using the lancing device 10 to obtain blood samples from skin puncture, the user would first need to vertically aim the front assembly 29 of said lancing device 10 to a desired puncture site on the patient's skin. Said front assembly 29 may need to contact the puncture site. The user then pulls said rear member 20 to activate the movements of various parts and elements of said lancing device 10. When using said lancing device 10, the first flexible element 14, the second flexible element 17 and the third flexible element 19 are deformed into a compressed state or a relaxed state, by pulling or pushing from external force, respectively. "Compressed state" refers to the change in length in a flexible element that results from the direct pulling or pushing from external force, or the pulling or pushing from other parts and elements in said lancing device 10. When in the compressed state, the elastic potential energy generated by the compression may be stored in said flexible element. "Relaxed state" refers to the condition wherein the elastic potential energy of said flexible element is released. When deforming from a compressed state into a relaxed state, said first flexible element 14 and said second flexible element 17 may release said elastic potential energy, so that multiple parts contacting or coupling with said flexible elements 14, 17 and 19 are moved due to the force associated with said elastic potential energy. The relative positions of internal parts and elements in said lancing device 10 are thus changed.

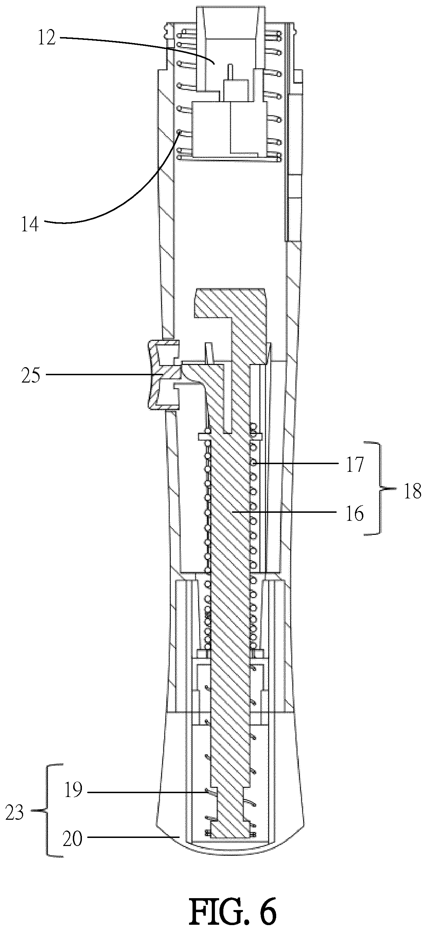

For various relative positions between internal parts and elements of the lancing device 10 when using said lancing device 10, please refer to Fig. 6 to Fig. 12.

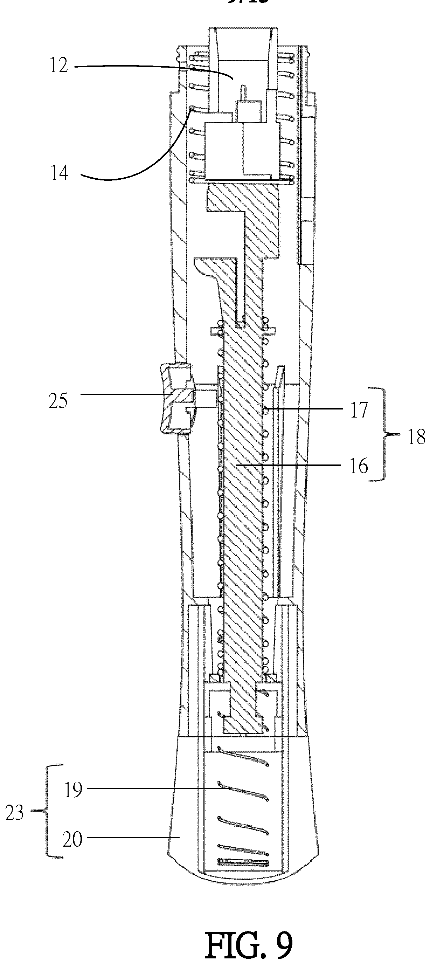

Referring to Fig. 9, it is a partial cross-sectional side view of the fourth state of the lancing device 10 provided by the present disclosure. Said fourth state is when the first flexible element 14, the second flexible element 17 and the third flexible element 19 are in relaxed states. Wherein, in said fourth state, the rod 16 in the impact assembly 18 does not contact with the trigger member 25, and said second flexible element 17 is in a relaxed state, and said first element 14 is in a relaxed state, and said impact assembly 18 does not contact with the lancet support assembly 12.

When the lancing device 10 is in the fourth state, the user may pull the rear member 20 in a direction that is away from the patient's skin. The third flexible element 19 is coupled to said rear member 20. Said third flexible element 19 may be deformed from a relaxed state into a compressed state. Said compressed state of said third flexible element 19 activates said second flexible element 17 in the impact assembly 18, so that the rod 16 will move in a direction that is away from the patient's skin. Above deformation of said third flexible element 19 and said second flexible element 17 lead said lancing device 10 into the first state.

Referring to Fig.6, it is a partial cross-sectional side view of the first state of the lancing device 10 provided by the present disclosure. Said first state is a pre-triggered position of said lancing device 10. Wherein, in said first state, the rod 16 in the impact assembly 18 contacts with the trigger member 25, and said trigger member 25 protrudes slightly outward because of its contact with the rod 16, and the second flexible element 17 is in a compressed state, and the first flexible element 14 is in a relaxed state, and said impact assembly 18 does not contact with said lancet support assembly 12. When in said first state, the user stops pulling the rear member 20. Said rear member 20 will then move toward the direction of the patient's skin and return to its' initial position.

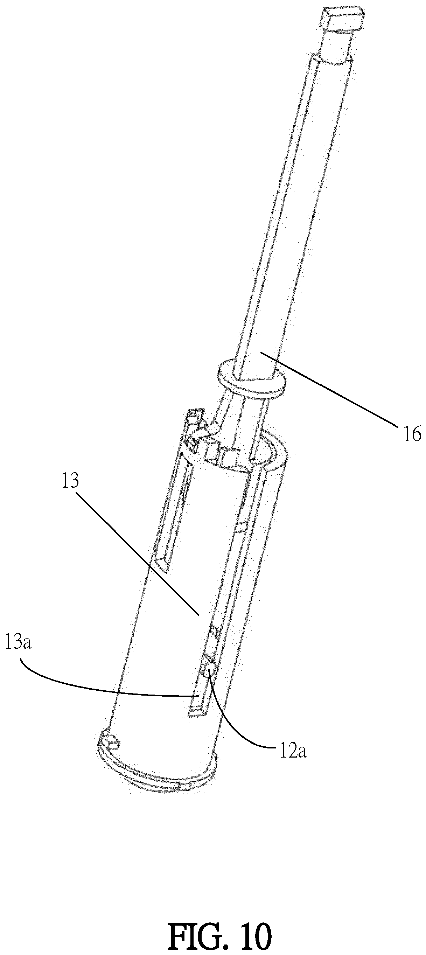

Referring to Fig. 10, it is a perspective view of the lancet support assembly 12, the sleeve 13 and the rod 16 in the first state of the lancing device 10. Said lancet support assembly 12 and said sleeve 13 are engaged with each other by the engaging part 12a of said lancet support assembly 12 and the sleeve engaging section 13a. Said sleeve engaging section 13a provides a sliding track for said engaging part 12a to move back-and-forth. Said lancet support assembly 12 and said rod 16 do not contact with each other when in said first state.

When the lancing device 10 is in the first state, the rod 16 in the impact assembly 18 contacts with the trigger member 25. Said trigger member 25 protrudes slightly outward because of its contact with said rod 16. The user may press said trigger member 25, so that said rod 16 of said impact assembly 18 may move toward the front end 11b of said housing 11. Said second flexible element 17 is deformed from a compressed state into a relaxed state. Above deformation of said second flexible element 17 and movement of said rod 16 leads said lancing device 10 into the second state.

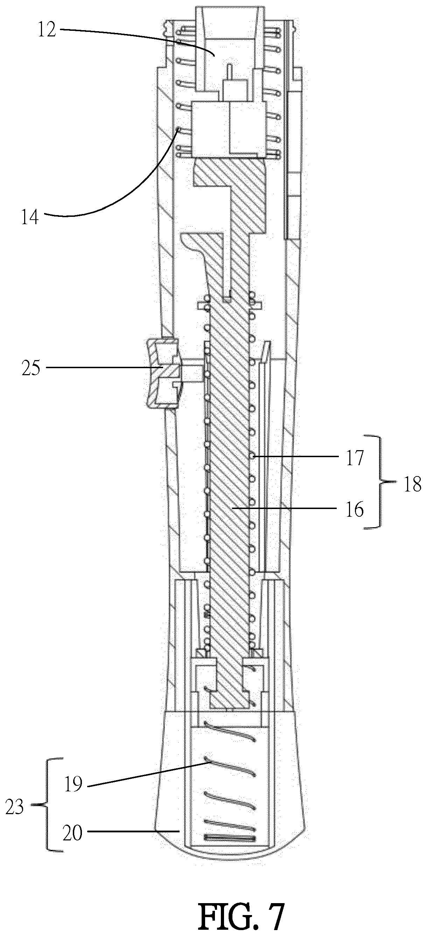

Referring to Fig. 7, it is a partial crosss-sectional side view of the second state of the lancing device 10 provided by the present disclosure. Wherein, in said second state, the rod 16 in the impact assembly 18 does not contact with the trigger member 25, and the second flexible element 17 is in a compressed state, and the first element 14 is in a relaxed state, and said rod 16 in said impact assembly contacts with the lancet support assembly 12.

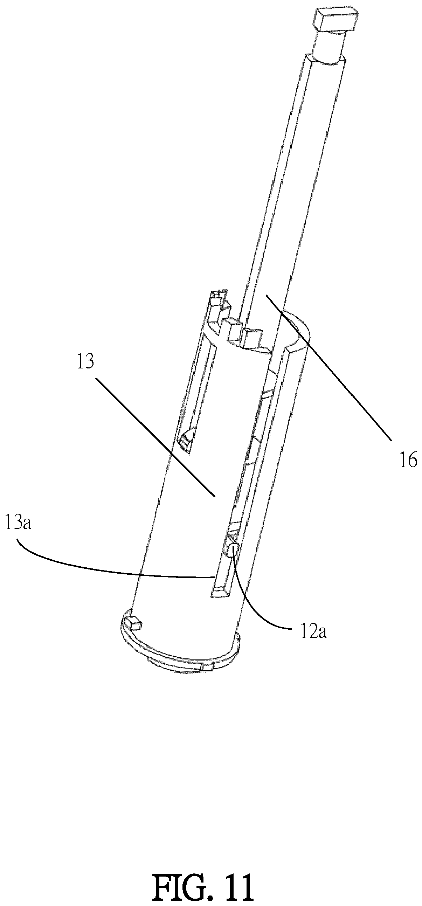

Referring to Fig. 11, it is a perspective view of the lancet support assembly 12, the sleeve 13 and the rod 16 in the second state of the lancing device 10. Said lancet support assembly 12 and said rod 16 contact with each other when in said second state.

When the lancing device 10 is in the second state, the rod 16 in the impact assembly 18 collides with the lancet support assembly 12. The kinetic energy of said rod 16 has been transferred to said lancet support assembly 12, so that said lancet support assembly 12 would move toward the patient's skin and outward of the housing 11. With collision between said rod 16 and said lancet support assembly 12, said first flexible element 14 is deformed from a relaxed state into a compressed state. Above deformation of said first flexible element 14 and movement of said lancet support assembly 12 leads said lancing device 10 into the third state.

The collision between the rod 16 and the lancet support assembly 12 can be generally supported by the principle of momentum conservation. If the speed of movement of said rod 16 remains constant and the mass of said rod 16 is significantly larger than the mass of said lancet support assembly 12, then the speed of said lancet support assembly 12 will be significantly faster than the speed of said rod 16 after the collision. Because the lancet 30 is installed on said lancet support assembly 12, so the faster said lancet support assembly 12 moves, the faster said lancet 30 moves. The faster movement speed of said lancet 30 will reduce the pain felt by the patient. For said lancet support assembly 12 to move faster toward the patient's skin, the overall mass of said impact assembly 18 should be larger than said lancet support assembly 12. Wherein, the mass of said rod 16 in said impact assembly 18 is larger than the mass of said lancet support assembly 12.

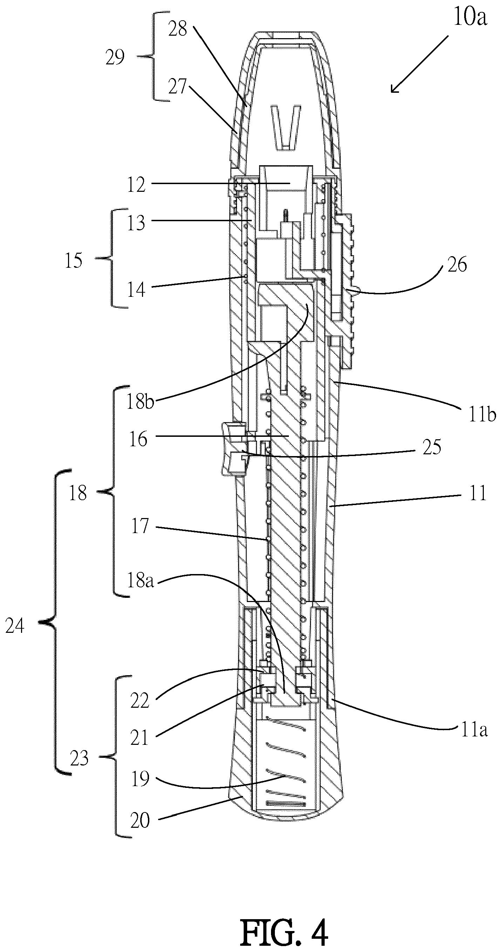

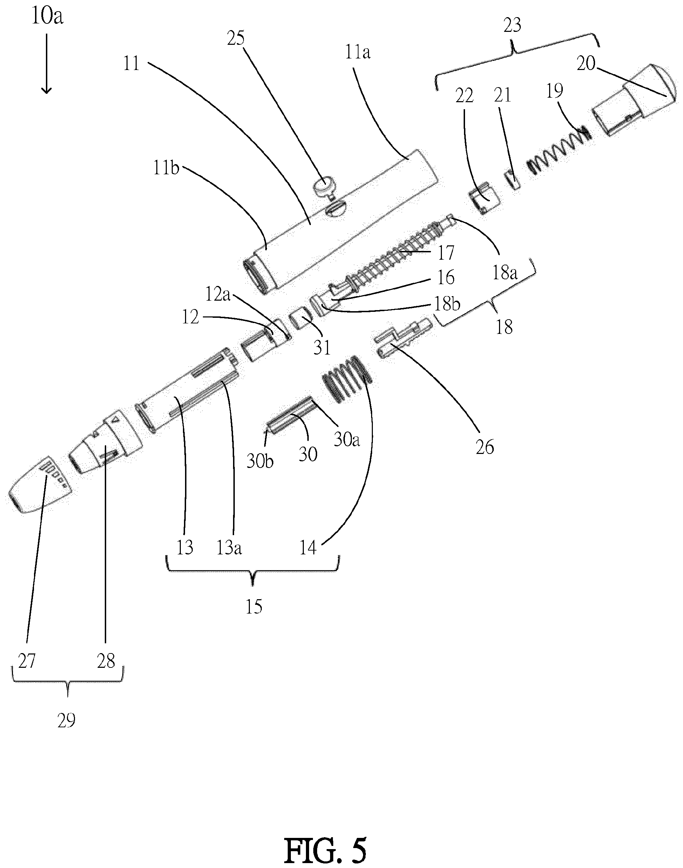

In order to amplify the effect of the collision, it would be helpful to concentrate the mass of said impact assembly 18 to its face of contact with said lancet support assembly 12. Referring to Fig. 4 and Fig. 5, Fig. 4 is a cross-sectional side view and Fig.5 is an exploded view of the lancing device 10a provided by the present disclosure. Fig. 4 and Fig. 5 further comprise a chunk member 31. Inside said lancing device 10a, the end of the rod 16 of the impact assembly 18 is coupled to said chunk member 31. Preferably, the mass of said chunk member 31 is larger than the mass of the lancet support assembly 12.

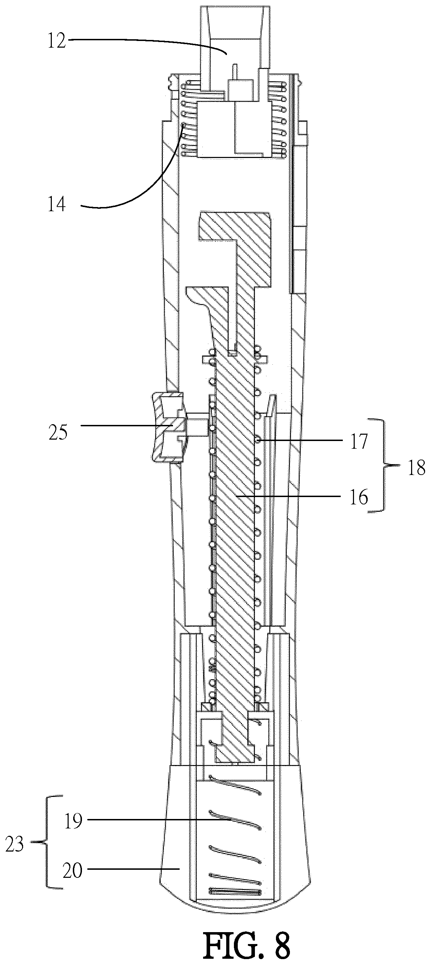

Referring to Fig. 8, it is a partial cross-sectional side view of the third state of the lancing device 10 provided by the present disclosure. Said second state and said third state is the triggered position of said lancing device 10. Wherein, in said third state, the rod 16 in the impact assembly 18 does not contact with the trigger member 25, and the second flexible element 17 is in a relaxed state, and the first flexible element 14 is in a compressed state. Because the lancet support assembly 12 receives the impact from said rod 16 in said second state, so that said lancet support assembly 12 moves toward the patient's skin. Above movement of said lancet support assembly 12 prevents said impact assembly 18 from contacting with said lancet support assembly 12.

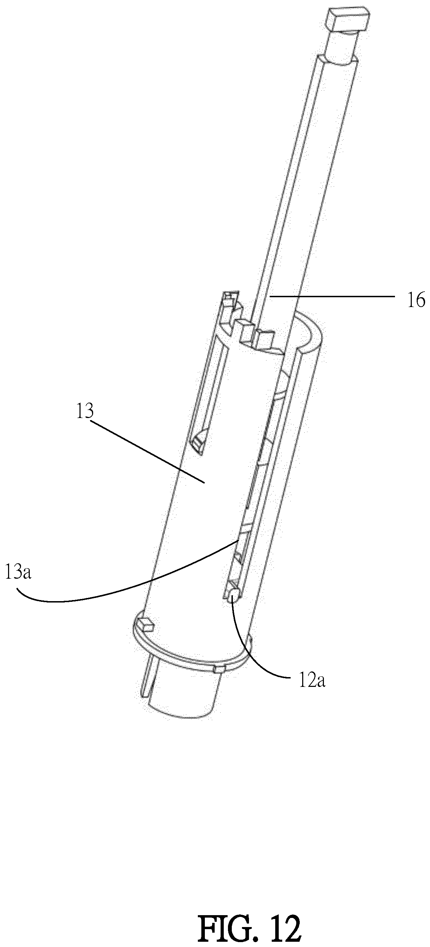

Referring to Fig. 12, it is a perspective view of the lancet support assembly 12, the sleeve 13 and the rod 16 in the third state of the lancing device 10. Said lancet support assembly 12 slides toward the patient's skin via the engagement between the engaging part 12a of said lancet support assembly 12 and the sleeve engaging section 13a. Said lancet support assembly 12 and said rod 16 does not contact with each other in said third state.

Referring to Fig. 10, Fig. 11 and Fig. 12. The lancet support assembly 12 has at least two engaging part 12a, and the sleeve 13 has at least two sleeve engaging sections 13a. So that said lancet support assembly 12 and said sleeve 13 may engage with each other and said lancet support assembly 12 may slide back-and-forth. If said engaging part 12a of said lancet support assembly is a protrusion, said sleeve engaging section 13a can be a notch or a protrusion that is specifically adapted to the protrusion shape of said engaging part 12a, in order to be engaged with said engaging part 12a. If said engaging part 12a of said lancet support assembly is a notch or concave member, said sleeve engaging section 13a can be a protrusion that specifically adapted to the concave or notch shape of said engaging part 12a, in order to be engaged with said engaging part 12a. If said engaging part 12a of said lancet support assembly and said sleeve engaging section 13a are both notches, another stick member may be used to insert through said engaging part 12a and said sleeve engaging section 13a to combine above parts, and aid lancet support assembly 12 to slide back-and-forth.

When the lancing device 10 is in the third state, the first flexible element 14 is in a compressed state. Said first flexible element 14 is deformed from a compressed state into a relaxed state due to its' elastic potential energy. Thus, the lancet support assembly 12 moves in a direction that is away from the patient's skin. Above deformation of said first flexible element 14 and movement of said lancet support assembly 12 lead said lancing device 10 into the fourth state.

The lancet 30 may comprise an engaging end 30a and a puncturing end 30b. Said puncturing end 30b is used to puncture through the skin. Said engaging end 30a may be coupled to said lancet support assembly 12. Said puncturing end 30b of lancet 30 can be of gauges of various sizes. Preferably, puncturing end 30b may comprise commercially available metal gauges , such as 28 Gauge, 30 Gauge or 33 Gauge.

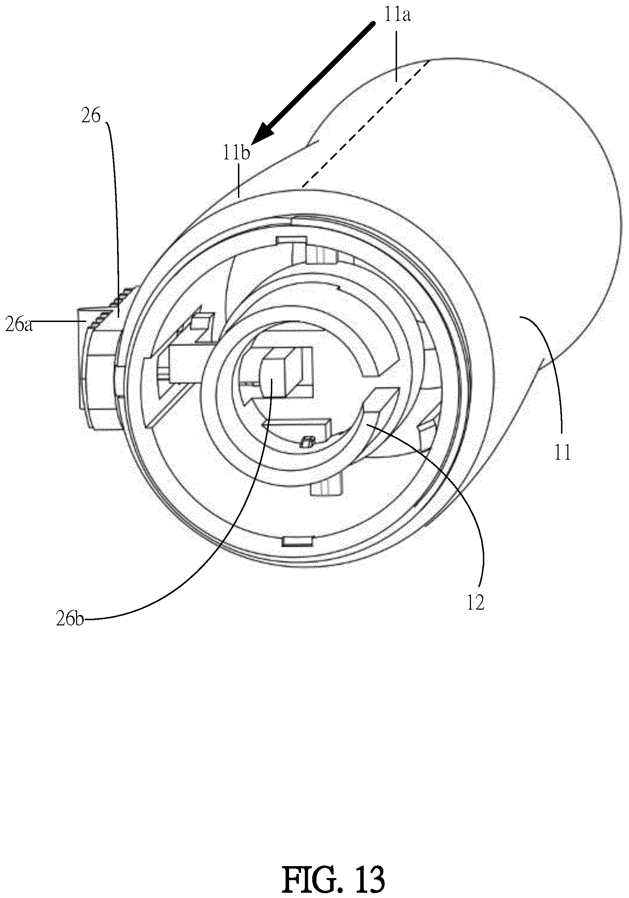

The present disclosure further directs to a dislodging and installing mechanism between the lancet 30 and the lancing device 10 or 10a. Referring to Fig. 13, it is the perspective view of the lancet support assembly 12, the housing 11 and the lancet ejection assembly 26 provided by said lancing device 10 in the present disclosure. The lancet ejection assembly 26 is located on the outer side of said housing 11, and a part of said lancet ejection assembly 26 is positioned through said housing 11 and engaged with said lancet support assembly 12. Said lancet ejection assembly 26 comprises a sliding part 26a of said lancet ejection assembly that is located on the outer side of said housing 11, and a protrusion part 26b of said lancet ejection assembly that is located inside said lancet support assembly 12. Said lancet ejection assembly 26 is used to dislodge said lancet 30 and said lancet support assembly 12. When dislodging said lancet 30, said lancet ejection assembly 26 may prevent the user from getting wounded. When dislodging said lancet 30, the user may slide said sliding part 26a toward the front end 11b of said housing 11. The above action results in said protrusion part 26b moving toward said front end 11b of said housing 11, then said lancet 30 that was originally installed on said lancet support assembly 12 will be ejected. The dislodgement of said lancet 30 and said lancet support assembly 12 is thus completed.

The material of the housing 11, the lancet support assembly 12, the sleeve 13, the rear member 20, the fixing member 22, the trigger member 25, the lancet ejection assembly 26, the outer front member 27 and the inner front member 28 can be POM plastic materials or ABS plastic materials. The material of the rod 16 and the chunk member 31 can be metal, plastic, or plastic mixed with metal particles. The first flexible element 14, the second flexible element 17 and the third flexible element 19 can be metal springs or other elastic components that can be deformed by external forces.

The lancing device of the present disclosure is described for exemplary and illustrative purposes only. Various modifications and changes may be made to the disclosed embodiments by persons skilled in the art without departing from the scope of the present disclosure as defined in the appended claims.

## Claims

1. A lancing device (10) that can be used at least once, comprising:
a housing (11), said housing comprises a front end (11b) and a rear end (11a), wherein, when in use, said front end points toward the puncture site and said rear end points away from said puncture site;
a lancet support assembly (12), said lancet support assembly is at least partially covered by said housing, and said lancet support assembly comprises at least two engaging parts;
a recovery assembly (15), said recovery assembly is at least partially covered by said housing, and said recovery assembly comprises a sleeve (13) and a first flexible element (14), and said sleeve is at said front end of said housing, and said sleeve can be engaged by said at least two engaging parts of said lancet support assembly, and said first flexible element is located on the outer side of said sleeve;
a loading assembly (24), said loading assembly is at least partially covered by said housing, and said loading assembly comprises a rear assembly (23) and an impact assembly (18), and said impact assembly comprises a first end (18a) and a second end (18b), and said rear assembly is coupled to said first end of said impact assembly, and said impact assembly and said rear assembly are positioned on the longitudinal axis of said lancing device, and said impact assembly further comprises a rod (16) and a second flexible element (17), and said rod and said second flexible element are positioned on the longitudinal axis of said lancing device;
a trigger member (25), said trigger member is located on the outer side of said housing.

2. The lancing device according to claim 1, wherein said lancing device has a first state, a second state, a third state and a fourth state;
wherein, in said first state, said impact assembly contacts with said trigger member, and said second flexible element is in a compressed state, and said first flexible element is in a relaxed state, and said impact assembly does not contact with said lancet support assembly;
wherein, in said second state, said impact assembly does not contact with said trigger member, and said second flexible element is in a relaxed state, and said first flexible element is in a compressed state, and said impact assembly contacts with said lancet support assembly;
wherein, in said third state, said impact assembly does not contact with said trigger member, and said second element is in a relaxed state, and said first flexible element is in a compressed state, and said impact assembly does not contact with said lancet support assembly;
wherein, in said fourth state, said impact assembly does not contact with said trigger member, and said second flexible element is in a relaxed state, and said first flexible element is in a relaxed state, and said impact assembly does not contact with said lancet support assembly.

3. The lancing device according to claim 2, wherein said rear assembly of said lancing device further comprises a third flexible element and a rear member, wherein said rear member and said third flexible element are positioned on the longitudinal axis of said lancing device, and said rear member at least partially covers said third flexible element.

4. The lancing device according to claim 3, when said lancing device is in said fourth state, said third flexible element can be deformed from a relaxed state into a compressed state, and the above deformation results in said second flexible element of said impact assembly being deformed from a relaxed state into a compressed state, so that said lancing device is transformed into said first state.

5. The lancing device according to claim 4, when said lancing device is in said first state, wherein said impact assembly contacts with said trigger member, and said trigger member can be pressed, and the above movement results in said second flexible element being deformed from a compressed state into a relaxed state, so that said rod of said impact assembly moves toward said front end of said housing, and said lancing device is transformed into said second state.

6. The lancing device according to claim 5, when said lancing device is in said second state, wherein said impact assembly contacts with said lancet support assembly, and said lancet support assembly moves outwardly, and the above movement results in said first flexible element being deformed from a relaxed state into a compressed state, and said lancing device is transformed into said third state.

7. The lancing device according to claim 6, when said lancing device is in said third state, wherein said first flexible element is in a compressed state, and said first element is deformed from a compressed state into a relaxed state, and the said lancing device is transformed into said fourth state.

8. The lancing device according to claim 1 to claim 7, wherein said rod of said impact assembly has a larger mass than said lancet support assembly.

9. The lancing device according to claim 8, wherein the material of said rod can be plastic, metal, or plastic mixed with metal particles.

10. The lancing device according to claim 1 to claim 7, wherein said impact assembly of said lancing device further comprises a chunk member, wherein said chunk member can be coupled to said rod, and said chunk member is located on said second end of said impact assembly.

11. The lancing device according to claim 10, wherein said chunk member has a larger mass than said lancet support assembly.

12. The lancing device according to claim 11, wherein said the material of said chunk member can be plastic, metal, or plastic mixed with metal particles.

13. The lancing device according to claim 2, wherein said lancing device further comprises a lancet, wherein said lancet can be removably installed on said lancet support assembly.

14. The lancing device according to claim 13, wherein said lancing device further comprises a lancet ejection assembly, wherein said lancet ejection assembly is located on the side of said housing and positioned through said housing to contact with said lancet support assembly, and said lancet ejection assembly can move parallel along the longitudinal axis of said lancing device, so that said lancet can be dislodged from said lancet support assembly.

15. The lancing device according to claim 14, wherein said lancet further comprises a puncturing end and an engaging end, and said engaging end can be engaged with said lancet support assembly, and said puncturing end can puncture through the skin.

16. The lancing device according to claim 15, wherein said lancing device further comprises a front assembly, wherein said front assembly contacts with said front end of said housing, and said front assembly covers said puncturing end of said lancet in said first state, said second state and said fourth state.

17. The lancing device according to claim 16, wherein said front assembly is used to adjust the depth of puncturing made by said lancet.

18. A method for obtaining blood sample by puncturing the skin, comprising:
(i) aiming the lancing device according to claim 16 to the puncture site on the skin;
(ii) pulling said rear assembly of said loading assembly in said lancing device, so that a movement of said rear assembly occurs, said movement results in the compression of said second flexible element, and said rod of said impact assembly moves toward said rear end of said housing, and said rod contacts with said trigger member;
(iii) pressing said trigger member, so that said second flexible element is deformed from a compressed state into a relaxed state, said deformation results in the movement of said rod toward said front end of said housing, and said rod collides with said lancet support assembly, so that a movement of said lancet support assembly occurs, and said first flexible element is compressed, and said puncturing end of said lancet on said lancet support assembly punctures the skin;
(iv) after said puncturing end of said lancet punctures the skin, said first flexible element is deformed from a compressed state into a relaxed state, so that said lancet support assembly moves toward said rear end of said housing and engages with said sleeve.

## Patentansprüche

1. Stechvorrichtung (10), die mindestens einmal verwendet werden kann, umfassend:
ein Gehäuse (11), wobei das Gehäuse ein vorderes Ende (11b) und ein hinteres Ende (11a) umfasst, wobei während der Verwendung das vordere Ende zur Einstichstelle hin zeigt und das hintere Ende von der Einstichstelle weg zeigt;
eine Lanzettenträgeranordnung (12),
wobei die Lanzettenträgeranordnung mindestens teilweise durch das Gehäuse bedeckt ist und die Lanzettenträgeranordnung mindestens zwei eingreifende Teile umfasst;
eine Rückgewinnungsanordnung (15),
wobei die Rückgewinnungsanordnung mindestens teilweise durch das Gehäuse bedeckt ist,
und die Rückgewinnungsanordnung eine Hülse (13) und ein erstes flexibles Element (14) umfasst
und die Hülse an dem vorderen Ende des Gehäuses ist und die Hülse durch die mindestens zwei eingreifenden Teile der Lanzettenträgeranordnung ergriffen werden kann und das erste flexible Element an der Außenseite der Hülse angeordnet ist;
eine Ladeanordnung (24),
wobei die Ladeanordnung mindestens teilweise durch das Gehäuse bedeckt ist, und die Ladeanordnung eine hintere Anordnung (23) und eine Aufprallanordnung (18) umfasst und die Aufprallanordnung ein erstes Ende (18a) und ein zweites Ende (18b) umfasst,
und die hintere Anordnung mit dem ersten Ende der Aufprallanordnung gekoppelt ist und die Aufprallanordnung und die hintere Anordnung auf der Längsachse der Stechvorrichtung positioniert sind und die Aufprallanordnung ferner eine Stange (16) und ein zweites flexibles Element (17) umfasst,
und die Stange und das zweite flexible Element auf der Längsachse der Stechvorrichtung positioniert sind;
ein Auslöseglied (25), wobei das Auslöseglied an der Außenseite des Gehäuses angeordnet ist.

2. Stechvorrichtung nach Anspruch 1, wobei die Stechvorrichtung einen ersten Zustand, einen zweiten Zustand, einen dritten Zustand und einen vierten Zustand hat;
wobei die Aufprallanordnung in dem ersten Zustand das Auslöseglied kontaktiert und das zweite flexible Element in einem komprimierten Zustand ist und das erste flexible Element in einem entspannten Zustand ist und die Aufprallanordnung die Lanzettenträgeranordnung nicht kontaktiert;
wobei die Aufprallanordnung in dem zweiten Zustand das Auslöseglied nicht kontaktiert und das zweite flexible Element in einem entspannten Zustand ist und das erste flexible Element in einem komprimierten Zustand ist und die Aufprallanordnung die Lanzettenträgeranordnung kontaktiert;
wobei die Aufprallanordnung in dem dritten Zustand das Auslöseglied nicht kontaktiert und das zweite flexible Element in einem entspannten Zustand ist und das erste flexible Element in einem komprimierten Zustand ist und die Aufprallanordnung die Lanzettenträgeranordnung nicht kontaktiert;
wobei die Aufprallanordnung in dem vierten Zustand das Auslöseglied nicht kontaktiert und das zweite flexible Element in einem entspannten Zustand ist und das erste flexible Element in einem entspannten Zustand ist und die Aufprallanordnung die Lanzettenträgeranordnung nicht kontaktiert.

3. Stechvorrichtung nach Anspruch 2, wobei die hintere Anordnung der Stechvorrichtung ferner ein drittes flexibles Element und ein hinteres Glied umfasst, wobei das hintere Glied und das dritte flexible Element auf der Längsachse der Stechvorrichtung positioniert sind und das hintere Glied mindestens teilweise das dritte flexible Element bedeckt.

4. Stechvorrichtung nach Anspruch 3, wobei, wenn die Stechvorrichtung in dem vierten Zustand ist, das dritte flexible Element von einem entspannten Zustand in einen komprimierten Zustand deformiert werden kann und die obige Deformation dazu führt, dass das zweite flexible Element der Aufprallanordnung von einem entspannten Zustand in einen komprimierten Zustand deformiert wird, sodass die Stechvorrichtung in den ersten Zustand transformiert wird.

5. Stechvorrichtung nach Anspruch 4, wobei, wenn die Stechvorrichtung in dem ersten Zustand ist, die Aufprallanordnung das Auslöseglied kontaktiert und das Auslöseglied gedrückt werden kann und die obige Bewegung dazu führt, dass das zweite flexible Element von einem komprimierten Zustand in einen entspannten Zustand deformiert wird, sodass sich die Stange der Aufprallanordnung zu dem vorderen Ende des Gehäuses hin bewegt und die Stechvorrichtung in den zweiten Zustand transformiert wird.

6. Stechvorrichtung nach Anspruch 5, wobei, wenn die Stechvorrichtung in dem zweiten Zustand ist, die Aufprallanordnung die Lanzettenträgeranordnung kontaktiert und sich die Lanzettenträgeranordnung auswärts bewegt und die obige Bewegung dazu führt, dass das erste flexible Element von einem entspannten Zustand in einen komprimierten Zustand deformiert wird und die Stechvorrichtung in den dritten Zustand transformiert wird.

7. Stechvorrichtung nach Anspruch 6, wobei, wenn die Stechvorrichtung in dem dritten Zustand ist, das erste flexible Element in einem komprimierten Zustand ist und das erste Element von einem komprimierten Zustand in einen entspannten Zustand deformiert wird und die Stechvorrichtung in den vierten Zustand transformiert wird.

8. Stechvorrichtung nach Anspruch 1 bis Anspruch 7, wobei die Stange der Aufprallanordnung eine größere Masse als die Lanzettenträgeranordnung hat.

9. Stechvorrichtung nach Anspruch 8, wobei das Material der Stange Kunststoff, Metall oder mit Metallpartikeln vermischter Kunststoff sein kann.

10. Stechvorrichtung nach Anspruch 1 bis Anspruch 7, wobei die Aufprallanordnung der Stechvorrichtung ferner ein Brockenglied umfasst, wobei das Brockenglied mit der Stange gekoppelt sein kann und das Brockenglied an dem zweiten Ende der Aufprallanordnung angeordnet ist.

11. Stechvorrichtung nach Anspruch 10, wobei das Brockenglied eine größere Masse als die Lanzettenträgeranordnung hat.

12. Stechvorrichtung nach Anspruch 11, wobei das Material des Brockenglieds Kunststoff, Metall oder mit Metallpartikeln vermischter Kunststoff sein kann.

13. Stechvorrichtung nach Anspruch 2, wobei die Stechvorrichtung ferner eine Lanzette umfasst, wobei die Lanzette entfernbar an der Lanzettenträgeranordnung installiert werden kann.

14. Stechvorrichtung nach Anspruch 13, wobei die Stechvorrichtung ferner eine Lanzettenauswurfanordnung umfasst, wobei die Lanzettenauswurfanordnung an der Seite des Gehäuses angeordnet ist und durch das Gehäuse hindurch positioniert ist, um die Lanzettenträgeranordnung zu kontaktieren und sich die Lanzettenauswurfanordnung parallel entlang der Längsachse der Stechvorrichtung bewegen kann, sodass die Lanzette von der Lanzettenträgeranordnung entfernt werden kann.

15. Stechvorrichtung nach Anspruch 14, wobei die Lanzette ferner ein Stechende und ein eingreifendes Ende umfasst und das eingreifende Ende in Eingriff mit der Lanzettenträgeranordnung gebracht werden kann und das Stechende durch die Haut hindurch stechen kann.

16. Stechvorrichtung nach Anspruch 15, wobei die Stechvorrichtung ferner eine vordere Anordnung umfasst, wobei die vordere Anordnung das vordere Ende des Gehäuses kontaktiert und die vordere Anordnung das Stechende der Lanzette in dem ersten Zustand, dem zweiten Zustand und dem vierten Zustand bedeckt.

17. Stechvorrichtung nach Anspruch 16, wobei die vordere Anordnung verwendet wird, um die durch die Lanzette hervorgerufene Stichtiefe einzustellen.

18. Verfahren zum Erhalten einer Blutprobe durch Stechen in die Haut, umfassend:
(i) Zielen mit der Stechvorrichtung nach Anspruch 16 auf die Stichstelle auf der Haut;
(ii) Ziehen der hinteren Anordnung der Ladeanordnung in der Stechvorrichtung, sodass eine Bewegung der hinteren Anordnung stattfindet, die Bewegung zu der Kompression des zweiten flexiblen Elements führt und sich die Stange der Aufprallanordnung zu dem hinteren Ende des Gehäuses hin bewegt und die Stange das Auslöseglied kontaktiert;
(iii) Drücken des Auslöseglieds, sodass das zweite flexible Element von einem komprimierten Zustand in einen entspannten Zustand deformiert wird, die Deformation zu der Bewegung der Stange zu dem vorderen Ende des Gehäuses hin führt und die Stange mit der Lanzettenträgeranordnung kollidiert, sodass eine Bewegung der Lanzettenträgeranordnung stattfindet und das erste flexible Element komprimiert wird und das Stechende der Lanzette an der Lanzettenträgeranordnung in die Haut sticht;
(iv) nachdem das Stechende der Lanzette in die Haut gestochen hat, wird das erste flexible Element von einem komprimierten Zustand in einen entspannten Zustand deformiert, sodass sich die Lanzettenträgeranordnung zu dem hinteren Ende des Gehäuses hin bewegt und in die Hülle eingreift.

## Revendications

1. Dispositif autopiqueur (10) qui peut être utilisé au moins une fois, comprenant :
un boîtier (11), ledit boîtier comprend une extrémité avant (11b) et une extrémité arrière (11a), dans lequel, lors de l'utilisation, ladite extrémité avant est orientée vers le site de piqûre et ladite extrémité arrière est orientée à l'opposé dudit site de piqûre;
un ensemble de support de lancette (12),
ledit ensemble de support de lancette est au moins partiellement recouvert par ledit boîtier, et ledit ensemble de support de lancette comprend au moins deux parties de mise en prise ;
un ensemble de récupération (15),
ledit ensemble de récupération est au moins partiellement recouvert par ledit boîtier, et ledit ensemble de récupération comprend un manchon (13) et un premier élément flexible (14), et ledit manchon est au niveau de ladite extrémité avant dudit boîtier, et ledit manchon peut se mettre en prise avec lesdites au moins deux parties de mise en prise dudit ensemble de support de lancette, et ledit premier élément flexible est situé sur le côté extérieur dudit manchon ;
un ensemble de chargement (24),
ledit ensemble de chargement est au moins partiellement recouvert par ledit boîtier,
et ledit ensemble de chargement comprend un ensemble arrière (23) et un ensemble d'impact (18),
et ledit ensemble d'impact comprend une première extrémité (18a) et une seconde extrémité (18b), et ledit ensemble arrière est couplé à ladite première extrémité dudit ensemble d'impact, et ledit ensemble d'impact et ledit ensemble arrière sont positionnés sur l'axe longitudinal dudit dispositif autopiqueur, et ledit ensemble d'impact comprend en outre une tige (16) et un deuxième élément flexible (17),
et ladite tige et ledit deuxième élément flexible sont positionnés sur l'axe longitudinal dudit dispositif autopiqueur ;
un organe de déclenchement (25),
ledit organe de déclenchement est situé sur le côté extérieur dudit boîtier.

2. Dispositif autopiqueur selon la revendication 1, dans lequel ledit dispositif autopiqueur présente un premier état, un deuxième état, un troisième état et un quatrième état ;
dans lequel, dans ledit premier état, ledit ensemble d'impact entre en contact avec ledit organe de déclenchement, et ledit deuxième élément flexible est dans un état comprimé, et ledit premier élément flexible est dans un état détendu, et ledit ensemble d'impact n'entre pas en contact avec ledit ensemble de support de lancette ;
dans lequel, dans ledit deuxième état, ledit ensemble d'impact n'entre pas en contact avec ledit organe de déclenchement, et ledit deuxième élément flexible est dans un état détendu, et ledit premier élément flexible est dans un état comprimé, et ledit ensemble d'impact entre en contact avec ledit ensemble de support de lancette ;
dans lequel, dans ledit troisième état, ledit ensemble d'impact n'entre pas en contact avec ledit organe de déclenchement, et ledit deuxième élément est dans un état détendu, et ledit premier élément flexible est dans un état comprimé, et ledit ensemble d'impact n'entre pas en contact avec ledit ensemble de support de lancette ;
dans lequel, dans ledit quatrième état, ledit ensemble d'impact n'entre pas en contact avec ledit organe de déclenchement, et ledit deuxième élément flexible est dans un état détendu, et ledit premier élément flexible est dans un état détendu, et ledit ensemble d'impact n'entre pas en contact avec ledit ensemble de support de lancette.

3. Dispositif autopiqueur selon la revendication 2, dans lequel ledit ensemble arrière dudit dispositif autopiqueur comprend en outre un troisième élément flexible et un organe arrière, dans lequel ledit organe arrière et ledit troisième élément flexible sont positionnés sur l'axe longitudinal dudit dispositif autopiqueur, et ledit organe arrière recouvre au moins partiellement ledit troisième élément flexible.

4. Dispositif autopiqueur selon la revendication 3, lorsque ledit dispositif autopiqueur est dans ledit quatrième état, ledit troisième élément flexible peut être déformé d'un état détendu en un état comprimé, et la déformation ci-dessus résulte en une déformation dudit deuxième élément flexible dudit ensemble d'impact d'un état détendu en un état comprimé, de sorte que ledit dispositif autopiqueur passe dans ledit premier état.

5. Dispositif autopiqueur selon la revendication 4, lorsque ledit dispositif autopiqueur est dans ledit premier état, dans lequel ledit ensemble d'impact entre en contact avec ledit organe de déclenchement, et ledit organe de déclenchement peut être enfoncé, et le déplacement ci-dessus résulte en une déformation dudit deuxième élément flexible d'un état comprimé en un état détendu, de sorte que ladite tige dudit ensemble d'impact se déplace vers ladite extrémité avant dudit boîtier, et ledit dispositif autopiqueur passe dans ledit deuxième état.

6. Dispositif autopiqueur selon la revendication 5, lorsque ledit dispositif autopiqueur est dans ledit deuxième état, dans lequel ledit ensemble d'impact entre en contact avec ledit ensemble de support de lancette, et ledit ensemble de support de lancette se déplace vers l'extérieur, et le déplacement ci-dessus résulte en une déformation dudit premier élément flexible d'un état détendu en un état comprimé, et ledit dispositif autopiqueur passe dans ledit troisième état.

7. Dispositif autopiqueur selon la revendication 6, lorsque ledit dispositif autopiqueur est dans ledit troisième état, dans lequel ledit premier élément flexible est dans un état comprimé, et ledit premier élément est déformé d'un état comprimé en un état détendu, et ledit dispositif autopiqueur passe dans ledit quatrième état.

8. Dispositif autopiqueur selon la revendication 1 à la revendication 7, dans lequel ladite tige dudit ensemble d'impact présente une masse plus importante que ledit ensemble de support de lancette.

9. Dispositif autopiqueur selon la revendication 8, dans lequel le matériau de ladite tige peut être du plastique, du métal ou du plastique mélangé à des particules métalliques.

10. Dispositif autopiqueur selon la revendication 1 à la revendication 7, dans lequel ledit ensemble d'impact dudit dispositif autopiqueur comprend en outre un organe de tronçon, dans lequel ledit organe de tronçon peut être couplé à ladite tige, et ledit organe de tronçon est situé sur ladite seconde extrémité dudit ensemble d'impact.

11. Dispositif autopiqueur selon la revendication 10, dans lequel ledit organe de tronçon présente une masse plus importante que ledit ensemble de support de lancette.

12. Dispositif autopiqueur selon la revendication 11, dans lequel ledit matériau dudit élément de tronçon peut être du plastique, du métal ou du plastique mélangé à des particules métalliques.

13. Dispositif autopiqueur selon la revendication 2, dans lequel ledit dispositif autopiqueur comprend en outre une lancette, dans lequel ladite lancette peut être installée de manière amovible sur ledit ensemble de support de lancette.

14. Dispositif autopiqueur selon la revendication 13, dans lequel ledit dispositif autopiqueur comprend en outre un ensemble d'éjection de lancette, dans lequel ledit ensemble d'éjection de lancette est situé sur le côté dudit boîtier et positionné à travers ledit boîtier pour entrer en contact avec ledit ensemble de support de lancette, et ledit ensemble d'éjection de lancette peut se déplacer parallèlement le long de l'axe longitudinal dudit dispositif autopiqueur, de sorte que ladite lancette puisse être délogée dudit ensemble de support de lancette.

15. Dispositif autopiqueur selon la revendication 14, dans lequel ladite lancette comprend en outre une extrémité de piqûre et une extrémité de mise en prise, et ladite extrémité de mise en prise peut se mettre en prise avec ledit ensemble de support de lancette, et ladite extrémité de piqûre peut piquer à travers la peau.

16. Dispositif autopiqueur selon la revendication 15, dans lequel ledit dispositif autopiqueur comprend en outre un ensemble avant, dans lequel ledit ensemble avant entre en contact avec ladite extrémité avant dudit boîtier, et ledit ensemble avant recouvre ladite extrémité de piqûre de ladite lancette dans ledit premier état, ledit deuxième état et ledit quatrième état.

17. Dispositif autopiqueur selon la revendication 16, dans lequel ledit ensemble avant est utilisé pour ajuster la profondeur de la piqûre effectuée par ladite lancette.

18. Procédé d'obtention d'un échantillon de sang par piqûre de la peau, comprenant :
(i) le pointage du dispositif autopiqueur selon la revendication 16 vers le site de piqûre sur la peau ;
(ii) la traction dudit ensemble arrière dudit dispositif de chargement dans ledit dispositif autopiqueur, de sorte qu'un déplacement dudit ensemble arrière se produise, ledit déplacement résulte en la compression dudit deuxième élément flexible, et ladite tige dudit ensemble d'impact se déplace vers ladite extrémité arrière dudit boîtier, et ladite tige entre en contact avec ledit organe de déclenchement ;
(iii) l'enfoncement dudit organe de déclenchement, de sorte que ledit deuxième élément flexible soit déformé d'un état comprimé en un état détendu, ladite déformation résulte en le déplacement de ladite tige vers ladite extrémité avant dudit boîtier, et ladite tige heurte ledit ensemble de support de lancette, de sorte qu'un déplacement dudit ensemble de support de lancette se produise, et que ledit premier élément flexible soit comprimé, et que ladite extrémité de piqûre de ladite lancette sur ledit ensemble de support de lancette pique la peau ;
(iv) après que ladite extrémité de piqûre de ladite lancette a piqué la peau, ledit premier élément flexible est déformé d'un état comprimé en un état détendu, de sorte que ledit ensemble de support de lancette se déplace vers ladite extrémité arrière dudit boîtier et se met en prise avec ledit manchon.
